# EUROPÄISCHE PATENTSCHRIFT

(11) **EP 0 381 091 B1**
(45) Veröffentlichungstag und Bekanntmachung des Hinweises auf die Patenterteilung: **31.05.1995**
(21) Anmeldenummer: 90101657.6
(22) Anmeldetag: 27.01.1990
(51) Int. Cl.: C12Q 1/25, C12Q 1/40, G01N 33/50, G01N 33/573

(54) **Verfahren zur Bestimmung eines Enzyms aus einem Isoenzymgemisch sowie hierfür geeigneter Testträger und dessen Verwendung**
Method for the determination of an enzyme from a mixture of isoenzymes, test carrier suited for the method and its use
Procédé pour mesurer l'activité d'une enzyme dans un mélange d'isoenzymes ainsi qu'un support de test approprié et son utilisation

(30) Priorität: 02.02.1989 DE 3903114
(43) Veröffentlichungstag der Anmeldung: 08.08.1990
(73) Patentinhaber: BOEHRINGER MANNHEIM GMBH, 68298 Mannheim (DE)
(72) Erfinder: Wilk, Hans-Erich, Dr. rer. nat., D-6143 Lorsch (DE); Rothe, Anselm, Dr. rer. nat., D-6943 Birkenau (DE); Schneider, Erich, Dr., D-6800 Mannheim 31 (DE)

(56) Entgegenhaltungen:
- EP-A- 0 150 309
- EP-A- 0 194 502
- EP-A- 0 196 731
- EP-A- 0 209 154
- EP-A- 0 293 649
- DE-A- 3 500 526
- CLINICAL CHEMISTRY, Band 31, Nr. 8, August 1985, Winston-Salem, NC (US); T.E. MIFFLIN et al., Seiten 1283-1288#
- CLINICAL CHEMISTRY, Band 28, Nr. 7, Juli 1982, Winston-Salem, NC (US); W.Y. HUANG et al., Seiten 1525-1527#

## Beschreibung

Die Erfindung betrifft ein Verfahren zur Bestimmung eines Enzyms aus einem Isoenzymgemisch in einer flüssigen Probe durch Inhibition der störenden Isoenzyme und Bestimmung des nichtinhibierten Enzyms.

Ein weiterer Gegenstand der Erfindung ist ein Testträger zur schnellen Bestimmung eines Enzyms aus einem Isoenzymgemisch in einer flüssigen Probe mittels Inhibition der störenden Isoenzyme und Bestimmung des nichtinhibierten Enzyms, enthaltend einen Probenaufgabe- und einen Auswertebereich sowie mehrere Testschichten.

Außerdem betrifft die Erfindung die Verwendung eines Testträgers zur Bestimmung eines Enzyms aus einem Isoenzymgemisch in einer flüssigen Probe.

Die Bestimmung von Enzymaktivitäten ist ein wichtiges Gebiet der medizinischen Diagnostik. So dient z. B. die Ermittlung der Aktivität bestimmter Enzyme in Körperflüssigkeiten, wie Blut, Serum, Plasma, Harn, Liquor, Speichel oder Duodenalsaft der Beurteilung, ob eine Erkrankung vorliegt oder nicht. Beispiele hierfür sind Lactat-Dehydrogenase (LDH), Creatin-Kinase (CK) oder Amylase. Auch Bestimmungen von Flüssigkeitsinhaltsstoffen, die selbst keine Enzyme sind, werden oft mittels Reaktionen durchgeführt, bei denen letztendlich ebenfalls eine Enzymaktivität gemessen wird, die ein Maß für die Konzentration des Inhaltsstoffes darstellt. Solche Substanzen können durch eine Kombination chemischer und enzymatischer Reaktionen oder durch enzymatische Reaktion allein bestimmt werden. Auch immunologische Bestimmungen münden oft in eine Enzymaktivitätsbestimmung. Beispiele hierfür sind z. B. die sogenannten "enzyme immuno assays" (EIA), bei denen Enzyme zur Markierung von Substanzen eingesetzt werden und deren gemessene Aktivität letztendlich ein Maß für die zu bestimmende Substanzkonzentration darstellt. Als Markierungsenzyme werden oft alkalische Phosphatase, Galaktosidase oder Peroxidase eingesetzt.

Ein Problem bei Enzymbestimmungen sind falsche oder diagnostisch nicht aussagekräftige Messungen aufgrund von verfälschenden Isoenzymen. Unter Isoenzymen werden Enzyme verstanden, die das gleiche Substrat zu dem gleichen Produkt umsetzen, die aber eine verschiedene Primärstruktur (Aminosäuresequenz) und/oder eine unterschiedliche Konformation aufweisen. Solche Enzyme können aus der gleichen oder selben Quelle stammen. Sie können aber auch unterschiedlichen Ursprungs sein.

Für die Erkennung von Organerkrankungen wird oft von dem Wissen Gebrauch gemacht, daß aus verschiedenen Organen organspezifische Enzyme in Körperflüssigkeiten gelangen. Bei Erkrankungen eines Organs können die üblicherweise in Körperflüssigkeiten, wie Blut, Plasma, Serum, Harn, Liquor, Speichel oder Duodenalsaft vorkommenden organspezifischen Enzyme in gegenüber dem normalen Maß veränderten Konzentrationen vorliegen. Isoenzyme verfälschen aber häufig eine eindeutige diagnostische Aussage. So existieren z. B. Isoenzyme des Muskel (M)- und Herz (H)-Typs der Lactat-Dehydrogenase (LDH), von denen nur der H4-Typ eine Aussage über den Verlauf eines Herzinfarkts erlaubt. Von der Creatin-Kinase (CK) sind Isoenzyme des Muskel (MM)-, Hirn (BB)- und Herz (MB)-Typs bekannt. Nur die CK-MB-Variante ist relevant für die Herzdiagnostik. α-Amylase existiert als Pankreas (P)- und als Speichel (S)-Typ. Wichtig für die Diagnostik der Bauchspeicheldrüse ist P-α-Amylase.

Immunteste erlauben häufig nicht den Einsatz an sich geeigneter Markierungsenzyme für die Untersuchung unverdünnter Proben, weil endogene Enzyme, d. h. in der zu untersuchenden Probenflüssigkeit vorliegende Enzyme, eine isoenzymatische Aktivität aufweisen. So existieren in Blutproben endogene alkalische Phosphatase- und Peroxidase-Aktivitäten, die die Verwendung entsprechender isoenzymatischer Markierungsenzyme nicht erlauben. Auch zu β-Galactosidase aus E. coli, das oft als Markierungsenzym verwendet wird, gibt es ein Isoenzym in Körperflüssigkeiten, das insbesondere bei der Verwendung von Substraten mit niedrigem pK-Wert stört.

Zum Nachweis eines Enzyms aus einem Isoenzymgemisch gibt es bestimmte Ansätze, die in gewissem Rahmen erfolgreich sind. In der europäischen Patentanmeldung 0 150 309 wird vorgeschlagen, für die Bestimmung von Pankreas-Amylase neben Speichel-Amylase, das Speichel-Isoenzym mittels Antikörpern zu präzipitieren und abzutrennen oder mittels fixierter Antikörper das Speichel-Isoenzym aus der Probe zu entfernen. In beiden Fällen ist ein Trennschritt erforderlich, der die Analyse kompliziert. Der Zeitaufwand für die Bestimmung von Pankreas-Amylase neben Speichel-Amylase nach diesem Verfahren beträgt insbesondere wegen der zeitaufwendigen Inkubation der Probe mit den inhibierenden Antikörpern ca. eine halbe Stunde.

In Clinical Chemistry 28, 1525 - 1527 (1982) wird ein Verfahren beschrieben, bei dem zur Bestimmung von Pankreas-Amylase neben Speichel-Amylase, das störende Speichelenzym mittels eines aus Weizenkeimen isolierten Inhibitors inaktiviert wird. Dies erlaubt zwar eine Messung in homogener Lösung, für Lösungen mit hoher Amylase-Aktivität wird jedoch eine Verdünnung vor der Bestimmung empfohlen. Außerdem ist die Selektivität unbefriedigend. Auch bei der optimalen Inhibitorkonzentration bleiben etwa 13 % der Aktivität des Speichel-Enzyms erhalten, während die Aktivität des Pankreas-Enzyms auf etwa 81 % verringert wird. Der Zeitaufwand für die Durchführung des in der genannten Literaturstelle beschriebenen Verfahrens beträgt ca. 10 - 15 Minuten, wovon 5 Minuten für die Inhibition des Speichelenzyms aufgewendet werden.

In der deutschen Offenlegungsschrift Nr. 35 00 526 werden monoklonale Antikörper beschrieben, die in homogener Lösung Speichelamylase hemmen und Pankreas-Amylase unbeeinträchtigt lassen. Für die Durchführung des Verfahrens zur Bestimmung von Pankreas-Amylase neben Speichel-Amylase wird die Probeflüssigkeit mit dem das Speichel-Enzym inhibierenden Antikörper inkubiert und danach die verbleibende Amylase-Aktivität gemessen. Die Dauer der Inkubationszeit zur Inhibierung des störenden Isoenzyms beträgt zwischen 15 und 30 Minuten.

In der deutschen Offenlegungsschrift Nr. 35 25 926 wird beschrieben, daß für die Bestimmung von Pankreas-Amylase neben Speichel-Amylase nach Inhibierung des Speichel-Isoenzyms durch monoklonale Antikörper die notwendige Inkubationszeit für die Inhibition wesentlich verringert werden kann, wenn zur Inhibierung der Speichelamylase zwei Antikörper eingesetzt werden, von denen der erste allein eine Hemmung von weniger als 93 % und der zweite Antikörper zwar an das Enzym bindet, aber nur eine Hemmung von weniger als 5 % erreicht. Die Inkubationszeit für die Inhibierung von Speichelamylase kann so auf etwa 5 Minuten verkürzt werden.

Die vorstehend genannten Literaturstellen beschreiben ausschließlich Naßtests, das sind solche Verfahren, bei denen die Probe mit Lösungen oder Suspensionen der benötigten Reagenzien in einer Küvette, einem Röhrchen oder einem entsprechenden Flüssigkeitsbehältnis in Kontakt gebracht wird. Die genau dosierte Zugabe von Reagenzien zu der zu untersuchenden Probe und weitere gegebenenfalls erforderliche Schritte erfordert geübtes Untersuchungspersonal und sind deswegen und wegen der erforderlichen Tätigkeiten während des Bestimmungsvorgangs personal- und zeitintensiv. Demgegenüber enthalten trägergebundene Tests alle für die Durchführung eines Verfahrens notwendigen Reagenzien auf einem festen Träger so angeordnet, daß nach Aufgabe der zu untersuchenden Probe auf den Träger keine weiteren Handgriffe des Untersuchungspersonals an der Probenflüssigkeit notwendig sind. Clinical Chemistry 31, 1000 (1985) gibt ein Beispiel für einen trägergebundenen Isoenzymtest. Es wird dort ein Dünnfilmelement vorgestellt, mit dem Creatin-Kinase B neben Creatin-Kinase M bestimmt werden kann. Das Verfahren beruht auf der Inhibition des störenden Isoenzyms Creatin-Kinase M durch entsprechende Antikörper und Messung der verbleibenden Creatin-Kinase-Aktivität, die der Creatin-Kinase B zuzuordnen ist. Die für das Bestimmungsverfahren benötigten Reagenzien liegen auf dem Testträger vor und kommen mit der flüssigen Probe nach der Aufgabe auf den Testträger in Kontakt.

Der Creatin-Kinase-Isoenzymtest benötigt eine Inkubationszeit zur Inhibierung störenden Isoenzyms von 7 Minuten und er ist nicht mit Vollblut durchführbar.

Eine optimierte Version dieses trägergebundenen Isoenzymtests wird in Clinical Chemistry 32, 1130 (1986) beschrieben. Durch Optimierung der Konzentrationen der für das Bestimmungsverfahren benötigten Reagenzien ist es gelungen, die Inkubationszeit zur Inhibierung des störenden Isoenzyms auf 3,5 Minuten zu senken. Insgesamt werden für die Bestimmung des interessierenden Enzyms mehr als 5 Minuten benötigt.

Wie der Stand der Technik zeigt, sind Verfahren zur Bestimmung eines Enzyms aus einem Isoenzymgemisch in einer flüssigen Probe durch Inhibition der störenden Isoenzyme und Bestimmung des nicht inhibierten Enzyms bekannt. Sie weisen jedoch Nachteile auf, da sie teilweise eine Abtrennung eines Probenbestandteils, z. B. des störenden Isoenzyms, erfordern, nicht auf unverdünnte Proben mit hoher Amylase-Aktivität oder nicht für Vollblut anwendbar sind. Ein genereller Nachteil der Verfahren des Standes der Technik ist, daß zur Hemmung des störenden Isoenzyms erhebliche Inkubationszeiten erforderlich sind, bevor die eigentliche Bestimmungsreaktion durchgeführt werden kann. Bisher ist kein Isoenzymtest bekannt, der in weniger als 5 Minuten abgeschlossen ist. Gerade für die moderne medizinische Diagnostik wird aber angestrebt, Bestimmungen möglichst schnell durchzuführen, sei es um die für die Behandlung von Notfällen dringend notwendigen Entscheidungen umgehend treffen zu können oder um innerhalb eines Arzttermins einen Patienten diagnostizieren und die entsprechende Therapie einleiten zu können.

Die Aufgabe der vorliegenden Erfindung bestand deshalb darin, eine Möglichkeit zu finden, wie ein Enzym aus einem Isoenzymgemisch möglichst schnell, auf jeden Fall aber in weniger als 5 Minuten bestimmt werden kann. Die Bestimmung sollte keinen separaten Abtrennschritt für das Isoenzym erfordern und sie sollte möglichst universell mit unverdünnten flüssigen Proben, insbesondere Körperflüssigkeiten, wie Harn, Speichel, Liquor, Duodenalsaft und vorzugsweise auch Blutplasma, Blutserum und Vollblut durchführbar sein. Vollblut und Plasma- oder Serumproben sollten vergleichbare Ergebnisse liefern. Außerdem sollte die Isoenzymbestimmung mit möglichst kleinen Probemengen durchgeführt werden können.

Diese Aufgabe wird durch den in den Patentansprüchen näher gekennzeichneten Gegenstand gelöst.

Insbesondere wird die Aufgabe durch ein Verfahren zur Bestimmung eines Enzyms aus einem Isoenzymgemisch in einer flüssigen Probe durch Inhibition der störenden Isoenzyme und Bestimmung des nicht inhibierten Enzyms, wobei das Isoenzymgemisch mit einer oder mehreren Substanzen kontaktiert wird, die die störenden Isoenzyme inhibieren, gelöst, das dadurch gekennzeichnet ist, daß
das Isoenzymgemisch in Gegenwart eines großporigen Materials mit einem möglichen Luftdurchlaß von mehr als 2000 l/m² sec mit einer oder mehreren Substanzen kontaktiert wird, die störende Isoenzyme inhibieren,
die die inhibierende(n) Substanz(en) enthaltende Probe in ein in direktem Kontakt mit dem großporigen Material stehenden engporiges Reaktionsmedium überführt wird, das überwiegend Poren besitzt, die kleiner als 25 »m sind und das störende Enzym dort inhibiert wird und
die Bestimmung des nicht inhibierten Enzyms in der resultierenden Flüssigkeit durchgeführt wird.

Außerdem wird die Aufgabe durch einen Testträger gelöst, der einen Probenaufgabe- und einen Auswertebereich sowie mehrere Testschichten enthält und der dadurch gekennzeichnet ist, daß
im Probenaufgabebereich ein großporiges Material mit einem möglichen Luftdurchlaß von mehr als 2000 l/m² sec eine oder mehrere Substanzen enthält, die die störenden Isoenzyme inhibieren,
hierzu ein engporiges Reaktionsmedium, das überwiegend Poren, die kleiner als 25 »m sind enthält in unmittelbarem, einen Flüssigkeitstransport aus dem großporigen Material ermöglichendem Kontakt steht und
im Auswertebereich eine oder mehrere Schichten angeordnet sind, die die für die Bestimmung des nicht inhibierten Enzyms mittels eines charakteristischen Signals notwendigen Substanzen enthalten und die in einem Flüssigkeitsübergang ermöglichenden Kontakt mit dem engporigen Reaktionsmedium stehen oder mit diesem in einen solchen Kontakt gebracht werden können.

Speziell wird die Aufgabe durch Verwendung eines vorstehend beschriebenen Testträgers zur Bestimmung eines Enzyms aus einem Isoenzymgemisch in einer flüssigen Probe gelöst.

Die vorliegende Erfindung beruht im wesentlichen darauf, daß überraschenderweise gefunden wurde, daß eine sehr schnelle Bestimmung eines Enzyms aus einem Isoenzymgemisch in einer flüssigen Probe durch Inhibition störender Isoenzyme und Bestimmung des nicht inhibierten Enzyms möglich ist, wenn die flüssige Probe - worunter auch eine von der Probe abgeleitete Flüssigkeit verstanden werden soll - mit der oder den gegen das oder die störenden Isoenzyme gerichteten Substanz(en) versetzt wird und die diese Substanz(en) enthaltende Flüssigkeit dann in ein engporiges Reaktionsmedium überführt wird, wo die Inhibition der störenden Isoenzyme im wesentlichen stattfindet. Die Inhibition ist innerhalb sehr kurzer Zeit, größenordnungsmäßig weniger als eine Minute, abgeschlossen. Anschließend kann die Bestimmung des nicht inhibierten Enzyms in bekannter Weise erfolgen.

Als flüssige Probe kann prinzipiell jede das zu bestimmende Enzym in einem Isoenzymgemisch enthaltende Flüssigkeit eingesetzt werden. Bevorzugt sind Körperflüssigkeiten, wie Blut, Plasma, Serum, Harn, Liquor, Speichel oder Duodenalsaft. Ganz besonders bevorzugt sind Blut, Plasma und Serum. Bei der Verwendung von Vollblut empfiehlt es sich in den meisten Fällen zelluläre Blutbestandteile, insbesondere Erythrozyten vor der Kontaktierung der Probe mit gegen störende Isoenzyme gerichteten Substanzen zu entfernen. Wenn Körperflüssigkeiten untersucht werden sollen, müssen diese zur Durchführung des erfindungsgemäßen Verfahrens nicht verdünnt werden.

Unter Inhibition wird im folgenden die vollständige Hemmung einer Enzymaktivität verstanden. Erfindungsgemäß versteht man unter einer vollständigen Hemmung eine Inhibition zu mehr als 90 %, vorzugsweise mehr als 95 %. Das heißt, das zu inhibierende Isoenzym darf nach Inhibition keine 10 %, vorzugsweise keine 5 % Restaktivität mehr aufweisen.

Für das erfindungsgemäße Verfahren sind zur Inhibierung von Isoenzymen alle Substanzen einsetzbar, die die vorgenannten Bedingungen erfüllen und die darüber hinaus die Aktivität des zu bestimmenden Enzyms im wesentlichen unbeeinflußt lassen. Die Kreuzreaktivität störende Isoenzyme inhibierender Substanzen mit dem zu bestimmenden Enzym sollte vorteilhafterweise weniger als 5 % betragen. Außerdem sollen die für das erfindungsgemäße Verfahren einsetzbaren inhibierenden Substanzen, die Bestimmung des nicht inhibierten Enzyms nicht negativ beeinflußen.

Prinzipiell sind für das erfindungsgemäße Verfahren alle inhibierenden Substanzen geeignet, die die vorstehenden Bedingungen erfüllen. Als besonders geeignet haben sich Antikörper gegen die störenden Isoenzyme erwiesen. Ganz besonders spezifisch inhibierend wirken monoklonale Antikörper. Derartige Antikörper sind bereits beschrieben oder ihre Herstellung ist dem Fachmann geläufig. Für das erfindungsgemäße Bestimmungsverfahren können Antikörper als solche oder ihre entsprechende inhibierende Eigenschaften aufweisenden Fragmente verwendet werden. Unter dem Begriff "Antikörper" werden hier daher auch Fragmente verstande.

Aus DE-A-35 00 526 sind monoklonale Antikörper bekannt, die Speichel-α-Amylase spezifisch hemmen. Hinterlegt sind solche Antikörper bei NCACC (National Collection of Animal Cell Culture, Porton Down, GB) unter den Nummern (99D12) 84122003 und (89E2) 84122004. Solche Antikörper sind vorteilhaft dazu geeignet, nach dem erfindungsgemäßen Verfahren Pankreas-α-Amylase neben Speichel-α-Amylase zu bestimmen. Das gleiche gilt für die in DE-A-35 25 926 beschriebene Kombination zweier Antikörper gegen Speichel-α-Amylase. Solche Antikörper sind bei NCACC (National Collection of Animal Cell Culture, Porton Down, GB) unter den Nummern (99D12) 84122003 und (89E2) 84122004 sowie 84111301 und 84111302 hinterlegt. Insbesondere die Kombination der gegen Speichel- -Amylase gerichteten monoklonalen Antikörper mit den Hinterlegungsnummern 84122003 und 84111301 oder 84122004 und 84111301 hat sich für das erfindungsgemäße Verfahren zur Bestimmung von Pankreas- -Amylase neben Speichel-α-Amylase hervorragend bewährt.

Für das erfindungsgemäße Verfahren ist es notwendig, daß das Isoenzymgemisch mit gegen die störenden Isoenzyme gerichteten inhibierenden Substanzen kontaktiert wird. Dies ist so zu verstehen, daß die Substanzen zu dem Isoenzymgemisch gegeben werden oder umgekehrt. Vorteilhaft ist es, wenn das Isoenzymgemisch zu der inhibierenden Substanz gegeben wird und diese so aufnimmt, daß ein homogenes Medium gebildet wird. "Homogenes Medium" bedeutet, daß die Substanz in inniger Vermischung mit dem Isoenzymgemisch vorliegt. Vorteilhafterweise ist die Substanz nach Kontaktierung durch das Isoenzymgemisch in der Flüssigkeit der Probe gelöst.

Die Inhibition des störenden Isoenzyms kann teilweise bereits während des Kontaktierens des Isoenzymgemischs mit der inhibierenden Substanz erfolgen. Um eine schnelle und vollständige Inhibition zu erreichen, muß die die inhibierende Substanz enthaltende Probe aber möglichst schnell in ein engporiges Reaktionsmedium überführt werden, wo die Inhibition schnell und vollständig stattfindet. Vorteilhafterweise wird die Probe innerhalb von weniger als einer Minute, vorzugsweise innerhalb weniger Sekunden nach Kontaktierung mit der inhibierenden Substanz in das engporige Reaktionsmedium eingebracht. Dort werden störende Isoenzyme in sehr kurzer Zeit, in der Regel in einer Minute oder weniger inhibiert.

Die Bestimmung der verbleibenden Enzymaktivität, die der Aktivität an dem zu bestimmenden Enzym entspricht, kann dann durch Zugabe der notwendigen Reagenzien innerhalb des engporigen Reaktionsmediums erfolgen oder die Probe kann aus dem engporigen Reaktionsmedium entfernt und außerhalb dieses Materials auf das zu bestimmende Enzym untersucht werden. Die Bestimmung des nicht inhibierten Enzyms erfolgt mittels bekannter Substrate nach bekannten Methoden. Für die Bestimmung von Pankreas-α-Amylase können z. B. die in DE-A-35 00 526, DE-A-35 25 926, EP-A-0 150 309 und Fresenius Z. Anal. Chem. 324 (1986) 304 - 305 beschriebenen Verfahren entsprechend durchgeführt werden.

Besonders schnell und vorteilhaft läßt sich das erfindungsgemäße Verfahren trägergebunden durchführen. "Trägergebunden" heißt, daß alle für die Durchführung des Verfahrens notwendigen Reagenzien und Materialien auf einem inerten Träger, vorzugsweise einem inerten Kunststoffmaterial angeordnet sind. Die trägergebundene Durchführung des Verfahrens ermöglicht eine sehr schnelle Bestimmung des gewünschten Enzyms auch mit sehr kleinen Probenmengen.

Für das erfindungsgemäße Verfahren insbesondere in trägergebundener Form ist die störende Isoenzyme inhibierende Substanz auf ein großporiges Material so aufgebracht, daß sie bei Kontakt mit der flüssigen Probe abgelöst wird. Auf dieses so beladene großporige Material wird das Isoenzymgemisch aufgegeben. Die inhibierende Substanz wird abgelöst. Durch physikalische Kräfte, wie z. B. Schwerkraft oder Kapillarkräfte, vorzugsweise aber durch Kapillarkräfte wird dann die inhibierende Substanz enthaltende Flüssigkeit in das engporige Reaktionsmedium überführt. Dies kann indirekt über ein anderes Material, vorteilhafterweise aber direkt durch einen unmittelbaren Flüssigkeitstransport ermöglichenden Kontakt zwischen groß- und engporigem Material erfolgen.

Großporige Materialien im Sinne der Erfindung sind grundsätzlich alle Materialien, die ein so große Oberfläche aufweisen, daß eine für das erfindungsgemäße Verfahren ausreichende Menge inhibierender Substanz aufgebracht und diese bei Kontakt mit Flüssigkeit sehr schnell abgelöst werden kann und die einen schnellen Flüssigkeitsdurchtritt ermöglichen. Die Ablösung des Inhibitors von dem großporigen Material und der Flüssigkeitstransport in das engporige Reaktionsmedium darf nicht den geschwindigkeitsbestimmenden Schritt des Bestimmungsverfahrens darstellen. Bevorzugt werden Vliese oder Gewebe monofiler oder multifiler Webart aus quellbarem oder nicht quellbarem Material verwendet. Wegen ihres geringeren Flüssigkeitsrückhaltevermögens, das insbesondere bei der Untersuchung kleiner Probenmengen sehr wichtig ist, werden nicht quellbare Materialien besonders bevorzugt eingesetzt. Unter Flüssigkeitsrückhaltevermögen wird diejenige Flüssigkeitsmenge verstanden, die durch die Saugkraft des engporigen Reaktionsmediums nicht aus dem großporigen Material herausgesaugt werden kann und dort verbleibt. Vorzugsweise soll das Flüssigkeitsrückhaltevermögen weniger als 20 %, besonders bevorzugt weniger als 10 % des eingesetzten Probenvolumens betragen. Geeignete großporige Materialien sind bevorzugt aus Polyester. Ebenfalls geeignet sind Nylongewebe oder Mischgewebe aus Nylon und Polyester. Besonders geeignet sind großporige Materialien mit einem mit der Porengröße in Beziehung stehendem Luftdurchlaß von mehr als 2000 l/m² sec. Es wurde gefunden, daß unterhalb dieser Luftdurchlaßgrenze der Probendurchtritt durch das großporige Material nicht mehr schnell erfolgt, wie es erfindungsgemäß notwendig ist, sondern daß der Flüssigkeitstransport durch dieses Material und aus diesem Material heraus zum geschwindigkeitsbestimmenden Schritt wird. Bevorzugt wird als großporiges Material Gewebe mit einer Dicke zwischen 70 und 140 »m und einem Luftdurchlaß zwischen 2000 und 10000 l/m² sec, insbesondere solche Gewebe mit einer Dicke von 85 bis 105 »m und 4000 bis 7000 l/m² sec Luftdurchlaß eingesetzt. Geeignet sind auch Einzelfaden Endlosvliese in vergleichbarer Dicke und Porenweite.

Ein engporiges Reaktionsmedium im Sinne der Erfindung ist ein Material, dessen Oberfläche für die zu untersuchende flüssige Probe benetzbar ist und das Poren kleiner als 25 »m, vorzugsweise mit einer Größe zwischen 0,5 und 25 »m, besonders bevorzugt zwischen 1 und 10 »m besitzt. Die Untergrenze der Porengröße wird durch die Viskosität der unverdünnt einsetzbaren Proben, insbesondere derjenigen von Blut, Plasma oder Serum bestimmt. Wird diese untere Grenze unterschritten, wird die vollständige Aufnahme der Probe in das engporige Reaktionsmedium zum geschwindigkeitsbestimmenden Schritt des Bestimmungsverfahrens. Da - wie gefunden wurde - die Inhibition störender Enzyme innerhalb des engporigen Reaktionsmediums wesentlich schneller abläuft als auf der äußeren Oberfläche des Reaktionsmediums oder ohne ein solches engporiges Material, muß die Probenflüssigkeit möglichst schnell in die engen Poren eindringen können. Die Porenobergrenze ergibt sich daraus, daß oberhalb dieser Porengröße aufgrund der geringeren Kapillarkräfte ebenfalls die Aufnahmegeschwindigkeit der Probe so niedrig ist, daß Materialien mit Poren oberhalb dieser Grenze die Lösung der erfindungsgemäßen Aufgabe nicht mehr erlauben.

Für das erfindungsgemäße Verfahren sollte die Saugkapazität des engporigen Reaktionsmediums 5 bis 100 »l/cm² betragen. Die Dicke des Materials kann zwischen 80 und 1000 »m sein.

Für den bevorzugten Fall, daß die Bestimmung des nicht inhibierten Enzyms nicht in dem engporigen, sondern in einem anderen Reaktionsmedium durchgeführt werden soll, sind komprimierbare Materialien als engporiges Reaktionsmedium besonders vorteilhaft. Die auf ihre Restaktivität zu untersuchende Flüssigkeit kann dann leicht aus dem engporigen Reaktionsmedium in ein anderes Material überführt werden, in dem letzteres auf ersteres gedrückt wird, so daß durch diesen Druck ein Flüssigkeitstransport ermöglichender Kontakt hergestellt wird. Der Flüssigkeitstransport wird im wesentlichen durch physikalische Kräfte, wie Schwerkraft und/oder Kapillarkräfte bewirkt.

Sowohl für das großporige als auch für das engporige Material, das in dem erfindungsgemäßen Verfahren Anwendung findet, ist wichtig, daß es keine Bindungen, weder adsorptiver noch chemischer Art mit Probeninhaltsstoffen eingeht. In diesem Zusammenhang wird unter Probeninhaltsstoffen nicht nur die in der ursprünglichen zu untersuchenden Probe enthaltenen Substanzen verstanden, sondern auch Substanzen und Reagenzien, die während des Bestimmungsverfahrens in die Probe gelangten. Unerwünschte Bindungen können Anlaß zu verfälschten Meßergebnisse geben. Materialien, die als solche Bindungen mit Probeninhaltsstoffen in diesem Sinn eingehen würden, können gegebenenfalls so behandelt werden, daß sie die unerwünschte Eigenschaft der unspezifischen Bindung von Probeninhaltsstoffen verlieren. Solchermaßen behandelte Stoffe sind dann auch für das erfindungsgemäße Verfahren als engporige oder großporige Materialien einsetzbar. Maßnahmen zur Unterbindung unerwünschter unspezifischer Bindungen von Probeninhaltsstoffen an feste Materialien sind dem Fachmann aus dem Stand der Technik bekannt. Beispielsweise kann der unerwünschten unspezifischen Bindung von Probenproteinen an die Testträgermaterialien durch Behandlung dieser Materialien mit Albumin vorgebeugt werden.

Vorteilhaft geeignet als engporige Reaktionsmedien sind Gewebe, Vliese, Membranen oder Filme, die vorstehende Eigenschaften besitzen.

Brauchbare Materialien für das engporige Reaktionsmedium sind Membranen, deren Oberflächen gegebenenfalls so modifiziert wurden, daß keine unerwünschte Bindung von Probeninhaltsstoffen im vorgenannten Sinne eintritt. Solche Materialien, die zum Kauf angeboten werden, sind z. B. Loprodyne^{R} von Pall, Glengrove, New York, USA oder hydrophile Durapore^{R} von Millipore, Bedford, USA. Auch mittels inerter Proteine, wie z. B. Albumin abgesättigte Cellulose- oder Cellulosederivatmembranen sind verwendbar. Als Cellulosederivate kommen z. B. Celluloseester, Celluloseether oder Nitrocellulose in Frage. Auch gegen unspezifische Bindungen behandelte Nylonmembranen sind als engporige Reaktionsmedien in dem erfindungsgemäßen Verfahren einsetzbar.

Ebenso sind vorteilhaft geeignet Filme, die gegebenenfalls aufgrund entsprechender Inhaltsstoffe, wie z. B. Kieselgur, porös sind. Solche sogenannten offenen Filme sind z. B. beschrieben in EP-A-0 016 387.

Vliese auf Basis von Zellwolle, Glasfasern oder Kunststoff, wie Nylon, Polyester, Polyethylen sind ebenfalls vorteilhaft einsetzbar.

Besonders gut geeignet als engporiges Reaktionsmedium sind Membranen mit Poren zwischen 3 und 7 »m, insbesondere von ca. 5 »m und Glasfaservliese mit Poren zwischen 1 und 10 »m, insbesondere zwischen 1 und 5 »m.

Zur Durchführung des erfindungsgemäßen Verfahrens in trägergebundener Form hat es sich als besonders vorteilhaft erwiesen, wenn das Porengrößenverhältnis für das großporige Material zu dem engporigen Reaktionsmedium zwischen 2 und 2000, bevorzugt 5 bis 500 und ganz besonders bevorzugt zwischen 10 und 100 liegt.

Zur Durchführung des erfindungsgemäßen Verfahrens in trägergebundener Form ist ein Testträger geeignet, der einen Probenaufgabe- und einen Auswertebereich sowie mehrere Testschichten enthält. Testträger sind häufig als längliche Teststreifen ausgebildet. Es sind jedoch auch Testträger bekannt, die als quadratische oder rechteckige Plättchen gestaltet sind. Im wesentlichen bestehen Testträger aus Testschichten, d. h. Materialien, die die für den durchzuführenden Test notwendigen Reagenzien enthalten. Es können wie im vorliegenden Fall aber auch Testschichten am Aufbau des Testträgers beteiligt sein, die keine Reagenzien tragen, sondern die beispielsweise aufgrund ihrer Materialstruktur die zu untersuchende Flüssigkeit von einer Testschicht zu einer anderen transportieren, die als Reaktionsmedium quasi wie ein Reaktionsgefäß miteinander reagierende Stoffe aufnehmen, oder die als Filter für bestimmte Probeninhaltsstoffe wirken.

Testträger im allgemeinen und insbesondere auch der erfindungsgemäße können in einen Probenaufgabe- und in einen Auswertebereich gegliedert werden. Der Probenaufgabebereich ist die Zone des Testträgers, wo die Probe aufgegeben wird. Der Auswertebereich ist die Zone, wo als Ergebnis der Untersuchung ein Signal gemessen wird, das ein Maß für den zu bestimmenden Parameter ist. Probenaufgabe- und Auswertebereich sind meist nicht identisch, sondern wie auch bei der vorliegenden Erfindung durch ein einen Flüssigkeitstransport ermöglichendes Medium miteinander verbunden.

Der erfindungsgemäße Testträger enthält im Probenaufgabebereich großporiges Material der zuvor beschriebenen Art, das eine oder mehrere Substanzen enthält, wie sie zuvor genannt wurden, die für die Bestimmung eines Enzyms aus einem Isoenzymgemisch störende Isoenzyme zu inhibieren in der Lage sind. Hieran anschließend ist ein engporiges Material der zuvor beschriebenen Art, das als Reaktionsmedium dient, so angeordnet, daß es mit dem großporigen Material in einem einen Flüssigkeitstransport aus diesem ermöglichenden Kontakt steht. Das engporige Material verbindet Probenaufgabe- und Auswertebereich des erfindungsgemäßen Testträgers und dient somit neben seiner Funktion als Reaktionsmedium zur Inhibition störender Isoenzyme auch zum Transport der zu untersuchenden flüssigen Probe aus dem Probenaufgabe- in den Auswertebereich.

Der Auswertebereich des erfindungsgemäßen Testträgers enthält eine oder mehrere Signalbildungsschichten. Die Signalbildungsschichten enthalten die für die Bestimmung des nicht inhibierten Enzyms notwendigen Reagenzien und sind so angeordnet, daß sie in einem einen Flüssigkeitsübergang ermöglichenden Kontakt mit dem engporigen Reaktionsmedium stehen oder mit diesem in einen solchen Kontakt gebracht werden können. Vorzugsweise ist sowohl mit dem Kontakt zwischen Signalbildungsschichten und engporigem Reaktionsmedium als auch mit dem Kontakt zwischen engporigem reaktionsmedium und großporigem Inhibitorträger jeweils ein flächiger Kontakt gemeint, so daß eine möglichst große Kontaktfläche hergestellt wird.

Bei Kontakt der zu untersuchenden Flüssigkeit mit den Nachweisreagenzien der Signalbildungsschichten wird ein nachweisbares Signal erzeugt, das ein Maß für die Menge des nicht inhibierten Enzyms darstellt. Die vorliegende Erfindung bezieht sich insbesondere auf solche Fälle, bei denen als nachweisbares Signal eine charakteristische Farbänderung erzeugt wird, wobei eine Farbbildung ebenfalls als Farbänderung (von farblos nach farbig) angesehen wird. Als Reagenzien für die Bestimmung des nicht inhibierten Enzyms aus dem Isoenzymgemisch können die zuvor für das Bestimmungsverfahren genannten eingesetzt werden.

Sind die für die signalbildende Reaktion notwendigen Reagenzien miteinander verträglich und beeinflußen sich nicht negativ, z. B. in Bezug auf ihre Stabilität, so können die Reagenzien zusammen in einer Schicht vorliegen. Gegebenenfalls kann es aber notwendig sein, daß die Bestimmungsreagenzien räumlich zu trennen und auf mehrere Schichten zu verteilen sind. In diesem Fall gibt es dann mehrere Signalbildungsschichten, die nur in der Summe die für die Bestimmungsreaktion insgesamt notwendigen Reagenzien enthalten.

Eine Ausführungsform eines bevorzugten erfindungsgemäßen Testträgers enthält diese Signalbildungsschichten so angeordnet, daß sie entweder vollflächig miteinander in Kontakt stehen oder doch miteinander so in Kontakt gebracht werden können. Ein solcher Kontakt muß bewirken, daß Flüssigkeit von einer Schicht in die andere übertreten kann.

Sind die für die Bestimmungsreaktion notwendigen Reagenzien in mehreren Signalbildungsschichten enthalten, müssen alle in einem Flüssigkeitsübergang ermöglichenden Kontakt mit dem engporigen Reaktionsmedium stehen oder mit diesem in einen solchen Kontakt gebracht werden können. In einer bevorzugten Ausführungsform des erfindungsgemäßen Testträgers steht nur eine von mehreren jeweils Teile der Bestimmungsreagenzien enthaltenden Schichten in direktem Kontakt mit dem engporigen Material oder kann mit diesem in einen direkten Kontakt gebracht werden. Die anderen den Rest der für die Bestimmungsreaktion notwendigen Reagenzien enthaltenden schichten stehen über diese vorgenannte Schicht in indirektem Kontakt mit dem engporigen Reaktionsmedium oder können mit diesem in einen solchen indirekten Kontakt gebracht werden. "Indirekter Kontakt" bedeutet hier, daß diese Schichten keinen direkten einen Flüssigkeitsübergang ermöglichenden Kontakt haben, sondern Flüssigkeit nur über eine dazwischen liegende Schicht übergehen kann.

Eine Signalbildungsschicht des erfindungsgemäßen Testträgers besteht aus einem Material, das alle oder einen Teil der für die Bestimmung des nicht inhibierten Enzyms notwendigen Reagenzien trägt. Dieses Reagenzträgermaterial kann prinzipiell aus allen hierfür üblichen einsetzbaren Materialien ausgewählt werden. Es können z. B. saugfähige oder quellbare, poröse oder nicht poröse Materialien oder auch solche, die sich bei Kontakt mit der zu untersuchenden Flüssigkeit darin auflösen, eingesetzt werden. Beispielhaft seien genannt Cellulose, Filterpapier, Kunstfaser- oder Glasfaservlies, Polyvinylester-, Polyamidfilme oder auch Filme aus z. B. Xanthan-Gum. Bevorzugt werden saugfähige, quellbare und/oder poröse Materialien, die einen guten Flüssigkeitsübergang von dem engporigen Reaktionsmedium in die Signalbildungsschicht(en) gewährleisten.

Das in der Signalbildungsschicht bzw. den Signalbildungsschichten erzeugte Signal kann im Falle einer Farbänderung sowohl visuell oder auch photometrisch bestimmt werden. Besonders bevorzugt ist die reflexionsphotometrische Vermessung des erzeugten Signals.

Für den Fall, daß der erfindungsgemäße Testträger zur Bestimmung eines Enzyms aus einen Isoenzymgemisch in Blut verwendet werden soll, besitzt er vorteilhafterweise im Probenaufgabebereich eine Schicht zur Abtrennung zellulärer Bestandteile aus Blut. Besonders vorteilhaft sind poröse Schichten, wie sie z. B. aus EP-A-0 045 476 bekannt sind, einsetzbar. Die Abtrennschicht ist in dem erfindungsgemäßen Testträger so angeordnet, daß die zu untersuchende Probe erst nach Durchlaufen dieser Schicht die großporige Schicht kontaktiert.

Der Vorteil der vorliegenden Erfindung ist vor allem darin zu sehen, daß es erstmals möglich wird, ein Enzym aus einem Isoenzymgemisch in weniger als 5 Minuten, vorzugsweise sogar in weniger als 3 Minuten zu bestimmen. Dies wird vor allem dadurch erreicht, daß störende Isoenzyme sehr schnell, vorzugsweise in einer Minute oder weniger in dem erfindungsgemäßen engporigen Reaktionsmedium inhibiert werden. Für die erfindungsgemäße Enzymbestimmung ist außerdem kein separater Abtrennschritt für störende Isoenzyme erforderlich. Es sind generell unverdünnte flüssige Proben, insbesondere Körperflüssigkeiten untersuchbar, wobei insbesondere als vorteilhaft hervorzuheben ist, daß Blut und Plasma- oder Serumproben vergleichbare Ergebnisse liefern. Schließlich reichen insbesondere bei der Verwendung erfindungsgemäßer Testträger Probenmengen von 30 »l oder weniger für die Bestimmung des gewünschten Enzyms.

Die Erfindung wird im folgenden anhand der in den Figuren schematisch dargestellten Ausführungsformen näher erläutert. Es zeigen
Fig. 1, 2 und 3 einen Querschnitt durch verschiedene Ausführungsformen eines erfindungsgemäßen Testträgers.
Fig. 4 zeigt die Seitenansicht einer bevorzugten Ausführungsform des erfindungsgemäßen Testträgers.

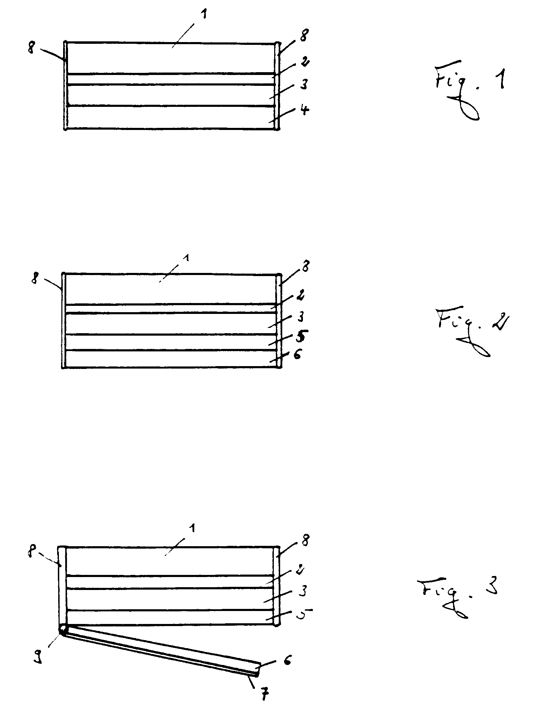

Fig. 1 zeigt im Querschnitt einen Testträger, bei dem alle Testschichten in einem einen Flüssigkeitsübergang ermöglichenden Kontakt stehen. Die Testschichten werden durch einen Rahmen (8) zusammengehalten. Der Rahmen (8) kann aus den unterschiedlichsten Materialien bestehen. Sie müssen nur die Aufgabe erfüllen, die Testschichten untereinander zusammenzuhalten. Geeignet ist z. B. ein Schmelzkleberstreifen oder auch ein Papprahmen, wie er z. B. von photographischen Diapositiven her bekannt ist.

Der Probenaufgabebereich besteht aus den schichten (1) und (2). (1) ist eine Schicht zur Abtrennung zellulärer Bestandteile aus Blut, insbesondere von Erythrozyten. Diese Blutzellenabtrennschicht ermöglicht es, das erfindungsgemäße Verfahren zur Bestimmung eines Enzyms aus einem Isoenzymgemisch in Vollblut durchzuführen, ohne daß vorher Blutzellen separat entfernt werden müssen. Als Material für die Blutzellenabtrennschicht kann prinzipiell jedes hierfür bekannte Material verwendet werden. Besonders bevorzugt sind jedoch Glasfaservliese, wie sie in EP-A-0 045 476 beschrieben sind. Sollen mit dem erfindungsgemäßen Testträger keine Vollblutproben untersucht werden, sondern nur solche Proben, die keine Entfernung zellulärer Bestandteile erfordern, dann ist auch keine Abtrennschicht 1 erforderlich. Um eine gleichmäßige Benetzung der Testschichten des Testträgers über ihre gesamte Fläche zu gewährleisten, empfiehlt es sich jedoch über den übrigen Testschichten eine Schicht (1) anzuordnen, die die aufgegebene Probenflüssigkeit zu spreiten in der Lage ist, auch wenn keine zellulären Probenbestandteile abzutrennen sind. Entsprechende Materialien für solche Spreitschichten sind aus dem Stand der Technik bekannt. Ein Glasfaservlies gemäß EP-A-0 045 476 bewirkt nicht nur eine abtrennung zellulärer Blutbestandteile, sondern auch eine gleichmäßige Befeuchtung der darunterliegenden Testschichten über deren gesamte Fläche. (2) ist eine Schicht aus großporigem Material, das die die störenden Isoenzyme inhibierende Substanz enthält. (3) ist das engporige Reaktionsmedium in dem störende Isoenzyme inhibiert werden. (4) ist die Signalbildungsschicht, die die für die Bestimmung des nicht inhibierten Enzyms notwendigen Reagenzien enthält und stellt den Auswertebereich des Testträgers dar.

Bei Verwendung des Testträgers der Fig. 1 zur Bestimmung eines Enzyms in einem Isoenzymgemisch wird die zu untersuchende Flüssigprobe auf die Schicht (1) aufgegeben, wo eine gleichmäßige Verteilung auf die gesamte Oberfläche der Testschicht erfolgt. Im Falle, daß es sich bei der zu untersuchenden Probe um Vollblut handelt, ist Schicht (1) ein Material, das außerdem zelluläre Blutbestandteile abzutrennen in der Lage ist. Die Flüssigkeit gelangt der Schwerkraft folgend und aufgrund kapillarer Kräfte in die großporige Schicht (2), wo sie die die störenden Isoenzyme inhibierende Substanz ablöst. Die mit diesen Substanzen versetzte Flüssigkeit wird sehr schnell in das engporige Reaktionsmedium (3) gesaugt. Für eine quantitative Bestimmung der Probe ist es erforderlich, daß das Flüssigkeitsrückhaltevermögen der Schicht (2) möglichst gering ist. In Schicht (3) erfolgt die Inhibition der die Bestimmung des gewünschten Enzyms störenden Isoenzyme, bevor die Flüssigkeit in die Signalbildungsschicht gelangt und dort ein Signal erzeugt, das ein Maß für die Menge an zu bestimmendem Enzym in der Probe darstellt. Vorzugsweise wird durch das zu bestimmende Enzym eine Farbänderung der Signalbildungsschicht verursacht. Diese Farbänderung kann von der der Probenaufgabeseite abgewandten Seite der Schicht 4 visuell oder photometrisch, besonders bevorzugt reflexionsphotometrisch, vermessen werden.

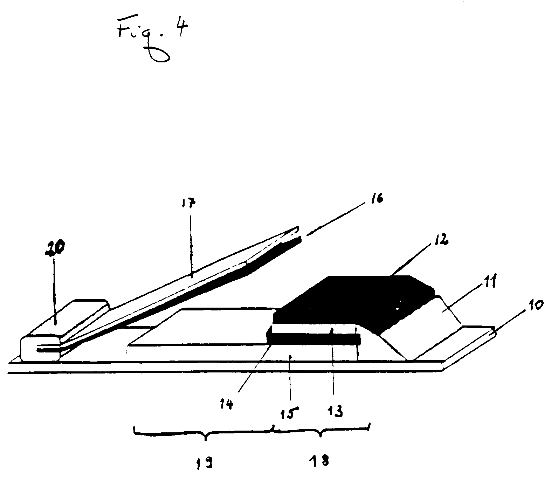

Fig. 2 unterscheidet sich von Fig. 1 dadurch, daß die dort gezeigte Ausführungsform des erfindungsgemäßen Testträgers nicht alle für die Bestimmung des zu messenden Enzyms notwendigen Reagenzien in einer einzigen Signalbildungsschicht enthält. Die Reagenzien sind hier vielmehr auf die Schichten (5) und (6) verteilt, welche beide zusammen den Auswertebereich darstellen. Miteinander unverträgliche Reagenzien können so in vorteilhafter Weise voneinander räumlich getrennt werden. Die zu untersuchende Flüssigkeit nimmt bei ihrem Durchtritt durch die Schicht (5) die für eine Reaktion mit Reagenzien der Schicht (6) notwendigen Reagenzien auf oder es laufen in Schicht (5) mit den dort vorliegenden Reagenzien erste Reaktionsschritte einer Reaktionssequenz ab, die dann mit den in Schicht (6) vorliegenden Reagenzien beendet wird.

In Fig. 3 ist der Auswertebereich wie eine Klappe ausgebildet. Schichten (5) und (6) enthalten die für die Bestimmungsreaktion notwendigen Reagenzien in räumlich getrennter Form. Schicht (6) ist zur besseren Handhabung auf eine durchsichtige, für die Bestimmungsreaktion inerte Folie (7) aufgebracht und mit einem beweglichen scharnierähnlichen Element (9) mit dem Rahmen (8) verbunden. Mit Hilfe der klappenartig angeordneten Schichten (5) und (6) ist es möglich, ein zeitlich genau definierte Bestimmungsreaktion zu starten. Ist nach Probenaufgabe die Flüssigkeit bis zur Schicht (5) durchgedrungen, und hat die dort befindlichen Reagenzien gelöst oder ist sie mit den dort befindlichen Reagenzien erste Reaktionsschritte einer aus mehreren Reaktionsschritten bestehenden Reaktionssequenz eingegangen, dann wird zu einem definierten Zeitpunkt durch Zuklappen Schicht (6) mit Schicht (5) in Kontakt gebracht und die Flüssigkeit geht in diese Schicht über. Die Bestimmungsreaktion wird zu ihrem Ende gebracht und kann visuell oder photometrisch über einen konkret bekannten Zeitraum durch die durchsichtige Folie (7) beobachtet werden.

Fig. 4 zeigt die bevorzugte Ausführungsform eines Testträgers in Streifenform. Auf einer steifen Basisfolie (10) sind mit Schmelzkleberstreifen (11) und (20) die für die Bestimmung notwendigen Testschichten befestigt. Im Probenaufgabebereich (18) sind unter einem Abdecknetz (12) aus Kunststoff, vorzugsweise Polyester, ein Vlies zur Abtrennung von zellulären Bestandteilen aus Blut (13), vorzugsweise ein Glasfaservlies und ein großporiger Inhibitorträger (14) angeordnet. Das engporige Reaktionsmedium (15) steht in einem einen Flüssigkeitsübergang ermöglichenden Kontakt mit dem Inhibitorträger (14) und reicht auf der Basisfolie (10) liegend aus dem Probenaufgabebereich (18) in den Auswertebereich (19). Die Signalbildungsschichten (16) und (17) sind mit einem Schmelzkleberstreifen (20) auf der steifen Basisfolie (10) so befestigt, daß sie sich im Auswertebereich befinden, das engporige Reaktionsmedium (15) jedoch nicht berühren.

Zur Durchführung des erfindungsgemäßen Verfahrens mittels des Testträgers der Fig. 4 wird die flüssige Probe, z. B. Vollblut auf das Abdecknetz (12) gegeben. Die flüssige Probe gelangt durch das Abdecknetz in Schicht (13), wo im Falle von Vollblut als Probe zelluläre Blutbestandteile zurückgehalten werden. Aufgrund der Schwerkraft und von Kapillarkräften gelangt die Probe in den Inhibitorträger (14), wo die die störenden Isoenzyme inhibierende Substanz abgelöst wird. Die Probe mit den Inhibitoren wird sehr schnell in das engporige Reaktionsmedium (15) gesaugt, wo die Inhibition sehr schnell erfolgt. Aufgrund von Kapillarkräften wird die Probe in dem engporigen Reaktionsmedium aus dem Probenaufgabebereich (18) in den Auswertebereich (19) transportiert. Nach Abschluß der Inhibition störender Isoenzyme werden die mit den zur Bestimmung des nicht inhibierten Enzyms versehenen Schichten (16) und (17) so auf das engporige Reaktionsmedium (15) gedrückt, daß die in diesen Schichten befindlichen Reagenzien mit der zu untersuchenden Flüssigkeit in Kontakt gelangen. Eine in Schicht (17) auftretende Farbänderung als Maß für die Menge an zu bestimmendem Enzym in der Probe kann visuell oder photometrisch, vorzugsweise reflexionsphotometrisch, in dieser Schicht von der der Basisfolie (10) gegenüberliegenden Seite vermessen werden.

Es ist selbstverständlich, daß die Dimensionen der Testträger und insbesondere die Dimensionen der einzelnen Testschichten dem zu untersuchenden Probenvolumen angepaßt sein müssen.

Die Erfindung wird anhand der folgenden Beispiele noch näher erläutert. Die Beispiele sollen jedoch keine Einschränkung des Erfindungsgegenstandes darstellen.

### Beispiel 1

### Vergleich verschiedener Testträger mit unterschiedlichen Materialkombinationen für Inhibitorträger und engporiges Reaktionsmedium

### A) Herstellung der Testträger

Es werden vier Testträger gemäß Fig. 4 hergestellt, wobei der Inhibitorträger (14) bei Modell 1 aus Polyester-Gewebe multi 14 normal der Porengröße 100 »m (Schweizer Seidengazefabrik, Thal, Schweiz), bei Modell 2 ebenfalls aus Polyester-Gewebe multi 14 normal der Schweizer Seidengazefabrik, Thal, Schweiz (Porengröße 100 »m), bei Modell 3 aus Langfaserpapier eines Flächengewichtes von etwa 17 g/m² und einer Porengröße zwischen etwa 50 - 300 »m (Schöller & Hösch, Bundesrepublik Deutschland) und bei Modell 4 aus einer Nylon-Membran mit 1,2 »m Porenweite (Pall, Glengrove, New York, USA) besteht und diese Materialien jeweils mit einer wässrigen Lösung imprägniert werden, die folgende Bestandteile enthält:
100 mM Phosphatpuffer, pH 7,0
50 mM Natriumchlorid
1 Gew.-% Crotein C (Croda, Cheshire, Großbritannien) und
soviel mg/ml Anti-Speichelamylase Antikörper der Hinterlegungsnummern 84122004 und 84111301 im Verhältnis 5:1, daß sich eine Menge von ca. 10 bis 12 »g Antikörper/cm² Gewebe ergibt.

Das engporige Reaktionsmedium (15) besteht in Modell 1 aus einem Glasfaservlies der Firma Binzer (Hatzfeld, Bundesrepublik Deutschland) mit einer Porengröße von etwa 1 bis 5 »m, in Modell 2 aus einer Nylonmembran mit einer Porengröße von 0,45 »m (Firma Pall, Glengrove, New York, USA), in Modell 3 aus einem Glasfaservlies der Firma Binzer (Hatzfeld, Bundesrepublik Deutschland) mit einer Porengröße von etwa 1 bis 5 »m und in Modell 4 ebenfalls aus einem Glasfaservlies der Firma Binzer (Hatzfeld, Bundesrepublik Deutschland) mit einer Porengröße von etwa 1 bis 5 »m.

Bei allen Modellen besteht die Signalbildungsschicht (16) aus einem Polyamidgewebe (NY20HC), Zürcher Beuteltuchfabrik, Zürich, Schweiz), das mit folgender wässriger Lösung imprägniert wurde:
5,8 g/l 2-Methoxy-4-morpholino-benzoldiazoniumchlorid x Zinkchlorid
20 Gew.-% Methanol,
so daß sich eine Menge von etwa 15,5 »g/cm² Diazoniumsalz ergibt.

Schicht (17) ist ein Reagenzfilm auf einer Kunststoffolie. Der Film wird so hergestellt, daß eine Lösung von:
3 g Keltrol F (Kelco, Dreieich, Bundesrepublik Deutschland)
15,5 g Natriumphosphat für Phosphatpuffer pH 7,0
0,6 g Natriumchlorid
1 MU α-Glucosidase und
25 g Indoxylmaltoheptaosid (Boehringer Mannheim GmbH, Mannheim, Bundesrepublik Deutschland)
in einem Liter Wasser bei einer Temperatur zwischen 0 und 4 °C auf eine Polycarbonatfolie der Firma Lonza, Weyl, Bundesrepublik Deutschland geschichtet wird, so daß sich eine Menge von 6 U/cm² Enzym bzw. 140 »g/cm² Indikator ergibt.

Als Erythrozytenabtrennvlies (13) wird für alle vier Modelle ein Glasfaservlies der Firma Binzer (Hatzfeld, Bundesrepublik Deutschland) mit einer Porengröße von 5 - 25 »m verwendet. Ebenfalls für alle vier Modelle wird ein Polyesternetz (12) (Zürcher Beuteltuchfabrik, Zürich, Schweiz) eingesetzt. Das Polyesternetz (12) und das Erythrozytenabtrennvlies (13) werden in 6 x 6 mm große Stücke, der Inhibitorträger (14) auf 6 x 7 mm, das engporige Reaktionsmedium (15) auf 16 x 6 mm und die Signalbildungsschichten (16) und (17) auf 13 x 6 mm zurechtgeschnitten, wie in Fig. 4 gezeigt, angeordnet und mit Schmelzkleberstreifen auf einer steifen Polystyrolfolie von 100 x 6 mm als Basisschicht (10) befestigt.

### B) Durchführung des Bestimmungsverfahrens

Blut- und Serumproben eines Spenders enthalten 42 U/l Pankreas-α-Amylase. Die Proben werden mit Speichel-Amylase auf insgesamt 8500 U/l Speichel-α-Amylase aufgestockt. Jeweils 30 »l Probe werden auf das Abdecknetz (12) der einzelnen Testträger der Modelle 1 bis 4 aufgegeben. Die Testträger werden in einem Reflexionsphotometer des Typs Reflotron^{R} der Firma Boehringer Mannheim GmbH, Mannheim, Bundesrepublik Deutschland, untersucht. Die Testzeit beträgt insgesamt 170 Sekunden. 1 Minute nach Probenaufgabe werden die schichten (17) und (16) auf das Reaktionsmedium gedrückt und so die Bestimmungsreaktion des nicht inhibierten Amylaseanteils gestartet. Die Farbbildung wird bei 567 nm reflexionsphotometrisch verfolgt. Es werden folgende Ergebnisse erhalten:

**Tabelle 1**

| Modell | eingesetzte Speichel-α-Amylase in U/l | Meßwert in U/l | Streuung (Variationskoeffizient) | Probenart |
|---|---|---|---|---|
| 1 | 8500 | 235 | 2,9 | Serum |
| 1 | 8500 | 231 | 3,5 | Blut |
| 2 | 8500 | 520 | 25,4 | Serum |
| 2 | 8500 | - | - | Blut |
| 3 | 8500 | 270 | 3,7 | Serum |
| 3 | 8500 | 225 | 4,8 | Blut |
| 4 | 8500 | 375 | 13,8 | Serum |
| 4 | 8500 | - | - | Blut |

Die Ergebnisse zeigen, daß lediglich für Modell 1 innerhalb der kurzen Zeit eine sehr gute Hemmung der Speichel-α-Amylase (mehr als 97 %) erreicht wurde und für Serum und Blut gut übereinstimmende Meßergebnisse erhalten werden. In Modell 2 sind die Poren des engporigen Reaktionsmediums offensichtlich so klein, daß die Probe nur sehr langsam eindringen kann und die Inhibition der Speichel-α-Amylase innerhalb der Testzeit nicht vollständig erfolgt. Mit Modell 3 ist die Inhibition der Speichel-α-Amylase gut. Die Ergebnisse für Serum und Blutproben weichen jedoch zu mehr als 16 % voneinander ab. Dies beruht offensichtlich auf dem Flüssigkeitsrückhaltevermögen von etwa 30 % des als großporigen Materials verwendeten Langfaserpapiers. Bei der Untersuchung gleicher Volumina an Blut und Serum ist zu berücksichtigen, daß bei Blut je nach Hämatokritwert nur bis zur Hälfte des eingesetzten Probenvolumens an Flüssigkeit zur Untersuchung zur Verfügung steht. Bei sehr kleinen Volumina, wie sie hier untersucht wurden, spielt dann das Flüssigkeitsrückhaltevermögen der einzelnen Schichtmaterialien eine große Rolle. Bei Modell 4 führt die kleine Porengröße des Inhibitorträgers (15) dazu, daß die Probe nur langsam in das engporige Reaktionsmedium gelangt und die Inhibition während der Testzeit nicht vollständig abläuft. Insbesondere bei Blut gelangt nur wenig Flüssigkeit in das engporige Reaktionsmedium, so daß die Flüssigkeit schon während der Meßzeit eingetrocknet ist und nicht vermessen werden konnte.

### Beispiel 2

### Testträger zum spezifischen Nachweis von Pankreas-α-Amylase

Es wird ein Testträger gemäß Fig. 4 hergestellt. Der Inhibitorträger (14) ist ein Polyester-Gewebe multi 14 normal (Schweizer Seidengazefabrik, Thal, Schweiz), das mit folgender wässriger Lösung imprägniert wird:
100 mM Phosphatpuffer, pH 7,0
50 mM Natriumchlorid
1 Gew.-% Crotein C (Croda, Cheshire, Großbritannien)
2 mg/ml Anti-Speichel-α-Amylase-Antikörper mit den Hinterlegungsnummern 84122004 und 84111301 im Verhältnis 5:1, so daß sich eine Menge von ca. 10 bis 12 »g Antikörper/cm² ergibt.

Als engporiges Reaktionsmedium (15) wird ein Glasfaservlies der Firma Binzer (Hatzfeld, Bundesrepublik Deutschland) mit einer Porengröße von etwa 1 bis 5 »m eingesetzt.

Die Signalbildungsschichten (16) und (17), das Abdecknetz (12) und das Erythrozytenabtrennvlies (13) entsprechen den in Beispiel 1 beschriebenen.

Die einzelnen Schichten werden wie in Beispiel 1 beschrieben zurechtgeschnitten und mit Schmelzkleberstreifen auf eine 100 x 6 mm große Polystyrolfolie zu einem Testträger gemäß Fig. 4 geklebt.

Auf das Abdecknetz (12) werden 30 »l Serum aufgegeben und der Teststreifen in einem Reflotron^{R}-Gerät der Firma Boehringer Mannheim GbmH, Mannheim, Bundesrepublik Deutschland, bei einer Meßwellenlänge von 567 nm reflexionsphotometrisch ausgewertet. Eine Minute nach Probenaufgabe werden die Signalbildungsschichten (16) und (17) auf das engporige Reaktionsmedium (15) gedrückt. Die Messung ist nach insgesamt 170 Sekunden ab Probenaufgabe beendet. Es werden folgende Ergebnise erhalten:

**Tabelle 2**

| eingesetzte -Amylase-Aktivität | | Meßwert für Pankreas-α-Amylase in U/l | Variationskoeffizient in % |
|---|---|---|---|
| Pankreas-Isoenzym in U/l | Speichel-Isoenzym in U/l | | |
| 43 | 8366 | 106 | 3,1 |
| 326 | 483 | 334 | 3,5 |
| 1260 | 25 | 1208 | 3,7 |

Die gefundenen Meßwerte für Pankreas-α-Amylase stimmen sehr gut mit den eingesetzten Amylase-Aktivitäten überein. In dem ersten Beispiel konnte der sehr große Überschuß an Speichel-Isoenzym zu 99 % inhibiert werden, so daß der gefundene Pankreas-Wert unter diesen Umständen ein hervorragendes Ergebnis darstellt.

### Beispiel 3

### Inhibition der Speichel-Amylase: Vergleich zum Naßtest

Ein Serum mit 4250 U/l Speichel-Amylase wurde untersucht und die erfindungsgemäße Methode mit dem kommerziell erhältlichen Naßtest PNP-Pankreas-Amylase (Boehringer Mannheim, Bundesrepublik Deutschland), der analoge inhibierende Antikörper verwendet, verglichen. Der Testträger wurde wie in Beispiel 2 beschrieben hergestellt. Die Messung wurde nach unterschiedlichen Inkubationszeiten mit den Antikörpern auf dem Teststreifen wie gewöhnlich durchgeführt. Es wurden Inkubationszeiten zwischen 5 und 120 Sekunden gewählt.

Die PNP-Pankreas-Amylase Methode wurde nach den Angaben des Herstellers durchgeführt, mit Ausnahme der Vorinkubationszeit, die von 30 bis 180 Sekunden variiert wurde.

Die resultierenden Ergebnisse sind in der Tabelle 3 zusammengestellt.

**Tabelle 3**

| Inkubationszeit in sec. | Restaktivität erfindungsgemäßes Verfahren in U/l | Inhibition in % | Restaktivität Naßchemie Methode in U/l | Inhibition in % |
|---|---|---|---|---|
| 5 | 211 | 95,0 | n.b.* | n.b.* |
| 30 | 134 | 96,9 | 383 | 90,8 |
| 60 | 122 | 97,1 | 207 | 95,1 |
| 120 | 106 | 97,5 | n.b.* | n.b.* |
| 180 | n.b.* | n.b.* | 142 | 96,7 |

| | | | | |
|---|---|---|---|---|
| *n.b. = nicht bestimmt | | | | |

Die erfindungsgemäße Methode garantiert eine Inhibition von 95 % nach 5 Sekunden Inkubationszeit und inhibiert > 97 % nach 1 Minute.

Der Naßtest benötigt fünf bis zehn mal länger (60 Sekunden und > 180 Sekunden), um die gleiche Inhibition zu bewirken.

## Patentansprüche

1. Verfahren zur Bestimmung eines Enzyms aus einem Isoenzymgemisch in einer flüssigen Probe durch Inhibition der störenden Isoenzyme und Bestimmung des nichtinhibierten Enzyms, wobei das Isoenzymgemisch mit einer oder mehreren Substanzen kontaktiert wird, die die störenden Isoenzyme inhibieren, dadurch gekennzeichnet, daß
das Isoenzymgemisch in Gegenwart eines großporigen Materials mit einem möglichen Luftdurchlaß von mehr als 2000 l/m² sec mit einer oder mehreren Substanzen kontaktiert wird, die störende Isoenzyme inhibieren,
die die inhibierende(n) Substanz(en) enthaltende Probe in ein in direktem Kontakt mit dem großporigen Material stehenden engporiges Reaktionsmedium überführt wird, das überwiegend Poren besitzt, die kleiner als 25»m sind und das störende Enzym dort inhibiert wird und
die Bestimmung des nicht inhibierten Enzyms in der resultierenden Flüssigkeit durchgeführt wird.

2. Verfahren gemäß Anspruch 1, dadurch gekennzeichnet, daß als Substanzen, die die störenden Isoenzyme inhibieren, Antikörper eingesetzt werden.

3. Verfahren gemäß einem der Ansprüche 1 oder 2, dadurch gekennzeichnet, daß zur Bestimmung von Pankreas-α-Amylase aus einem Gemisch von Pankreas- und Speichel-α-Amylase ein Antikörper oder eine Kombination von Antikörpern eingesetzt wird, der oder die Speichel-α-Amylase inhibiert.

4. Testträger zur schnellen Bestimmung eines Enzyms aus einem Isoenzymgemisch in einer flüssigen Probe mittels Inhibition der störenden Isoenzyme und Bestimmung des nicht inhibierten Enzyms, enthaltend einen Probenaufgabe- und einen Auswertebereich, sowie mehrere Testschichten, dadurch gekennzeichnet, daß
im Probenaufgabebereich ein großporiges Material mit einem möglichen Luftdurchlaß von mehr als 2000 l/m² sec eine oder mehrere Substanzen enthält, die die störenden Isoenzyme inhibieren,
hierzu ein engporiges Reaktionsmedium das überwiegend Poren, die kleiner als 25»m enthält in unmittelbarem, einen Flüssigkeitstransport aus dem großporigen Material ermöglichenden Kontakt steht und
im Auswertebereich eine oder mehrere Schichten angeordnet sind,, die die für die Bestimmung des nicht inhibierten Enzyms mittels eines charakteristischen Signals notwendigen Substanzen enthalten und die in einem Flüssigkeitsübergang ermöglichenden Kontakt mit dem engporigen Reaktionsmedium stehen oder mit diesem in einen solchen Kontakt gebracht werden können.

5. Testträger gemäß Anspruch 4, dadurch gekennzeichnet, daß das Verhältnis der Porengröße von großporigem Material und engporigem Reaktionsmedium größer ist als 2.

6. Testträger gemäß einem der Ansprüche 4 oder 5, dadurch gekennzeichnet, daß im Probenaufgabebereich eine Schicht zur Abtrennung zellulärer Bestandteile aus Blut so angeordnet ist, daß die zu untersuchende Probe erst nach Durchlaufen dieser Schicht die großporige Schicht kontaktiert.

7. Verwendung eines Testträgers gemäß einem der Ansprüche 4 bis 6 zur Bestimmung eines Enzyms aus einem Isoenzymgemisch in einer flüssigen Probe.

## Claims

1. Process for the determination of an enzyme from an isoenzyme mixture in a liquid sample by inhibition of the disturbing isoenzymes and determination of the non-inhibited enzyme, whereby the isoenzyme mixture is contacted with one or more substances which inhibit the disturbing isoenzymes, characterised in that the isoenzyme mixture is contacted, in the presence of a large-pored material with a possible air passage of mare than 2000 l/m² sec, with one or more substances which inhibit the disturbing isoenzymes, the sample containing the inhibiting substance(s) is transferred into a narrow-pored reaction medium, standing in direct contact with the large-pored material, which preponderantly possesses pores which are smaller than 25 »m and the disturbing enzyme is there inhibited and the determination of the non-inhibited enzyme is carried out in the resulting liquid.

2. Process according to claim 1, characterised in that antibodies are used as substances which inhibit the disturbing isoenzymes.

3. Process according to one of claims 1 or 2, characterised in that, for the determination of pancreatic α-amylase from a mixture of pancreatic and salivary α-amylase, an antibody or a combination of antibodies is used which inhibit the salivary α-amylase.

4. Test carrier for the rapid determination of an enzyme from an isoenzyme mixture in a liquid sample by means of inhibition of the disturbing isoenzymes and determination of the non-inhibited enzyme, containing a sample application and an evaluation region, as well as several test layers, characterised in that in the sample application region a large-pored material with a possible air passage of more than 2000 l/m² sac contains one or more substances which inhibit the disturbing isoenzymes, to this stands in contact a narrow-pored reaction medium which preponderantyl contains pores which are smaller than 25 »m making possible a liquid transport from the large-pored material and in the evaluation region are provided one or more layers which contain the substances necessary for the determination of the non-inhibited enzyme by means of a characteristic signal and which stand in contact with the narrow-pored reaction medium making possible a liquid transfer or can be brought into such contact with this.

5. Test carrier according to claim 4, characterised in that the ratio of the pore size of large-pored material and narrow-pored material is greater than 2.

6. Test carrier according to one of claims 4 or 5, characterised in that in the sample application region is so arranged a layer for the separation of cellular components from blood that the sample to be investigated is first contacted with the large-pored layer after passing through this layer.

7. Use of a test carrier according to one of claims 4 to 6 for the determination of an enzyme from an isoenzyme mixture in a liquid sample.

## Revendications

1. Procédé de détermination d'une enzyme à partir d'un mélange d'isoenzymes dans un échantillon liquide, par inhibition des isoenzymes perturbatrices et détermination de l'enzyme non inhibée, le mélange d'isoenzymes étant mis en contact avec une ou plusieurs substance(s) qui inhibe(nt) les isoenzymes perturbatrices, caractérisé en ce que le mélange d'isoenzymes est mis en contact, en présence d'un matériau à grands pores ayant une perméabilité à l'air supérieure à 2000 l/m²·sec, avec une ou plusieurs substance(s) qui inhibe(nt) les isoenzymes perturbatrices, l'échantillon contenant la ou les substance(s) inhibitrice(s) est transféré dans un milieu réactionnel à pores étroits, qui est en contact avec le matériau à grands pores et qui présente en proportion prépondérante des pores dont le diamètre est inférieur à 25 »m et l'enzyme perturbatrice y est inhibée et la détermination de l'enzyme non inhibée est effectuée dans le liquide obtenu.

2. Procédé selon la revendication 1, caractérisé en ce que comme substances inhibant les isoenzymes perturbatrices, on utilise des anticorps.

3. Procédé selon l'une des revendications 1 ou 2, caractérisé en ce que pour la détermination de l'α-amylase pancréatique dans un mélange d'α-amylases pancréatique et salivaire, on utilise un anticorps ou une combinaison d'anticorps qui inhibe(nt) l'α-amylase salivaire.

4. Support de test pour la détermination rapide d'une enzyme à partir d'un mélange d'isoenzymes dans un échantillon liquide, par inhibition des isoenzymes perturbatrices et détermination de l'enzyme non inhibée, contenant une zone d'application de l'échantillon et une zone d'évaluation, ainsi que plusieurs couches de test, caractérisé en ce que dans la zone d'application de l'échantillon, un matériau à grands pores, ayant une perméabilité à l'air supérieure à 2000 l/m²·sec, qui contient une ou plusieurs substance(s) inhibant les isoenzymes perturbatrices, ainsi qu'un mileu réactionnel à pores étroits, qui présente en proportion prépondérante des pores dont le diamètre est inférieur à 25 »m, sont en contact direct, permettant un transport de liquide à partir du matériau à grands pores, et dans la zone d'évaluation, se trouve(nt) une ou plusieurs couche(s), qui contiennent les substances nécessaires pour la détermination de l'enzyme non inhibée au moyen d'un signal caractéristique, et qui sont en contact permettant un transfert de liquide avec le milieu réactionnel à pores étroits, ou peuvent être amenées en un tel contact avec celui-ci.

5. Support de test selon la revendication 4, caractérisé en ce que le rapport de la taille des pores du matériau à grands pores à celle des pores du milieu réactionnel à pores étroits est supérieur à 2.

6. Support de test selon la revendication 4 ou 5, caractérisé en ce que dans la zone d'application de l'échantillon, une couche destinée à séparer les constituants cellulaires du sang est disposée de telle façon que l'échantillon à déterminer ne rencontre la couche à grands pores qu'après avoir traversé cette couche.

7. Utilisation d'un support de test selon l'une quelconque des revendications 4 à 6, pour la détermination d'une enzyme à partir d'un mélange d'isoenzymes dans un échantillon liquide.
